# EUROPEAN PATENT SPECIFICATION

(11) **EP 2 238 911 B1**
(45) Date of publication and mention of the grant of the patent: **28.11.2012**
(21) Application number: 10155770.0
(22) Date of filing: 08.03.2010
(51) Int. Cl.: A61B 8/08

(54) **Providing stress information in an ultrasound system**
Bereitstellung von Spannungsinformationen in einem Ultraschallsystem
Fourniture d'informations de stress dans un système à ultrasons

(30) Priority: 10.04.2009 KR 20090031063
(43) Date of publication of application: 13.10.2010
(73) Proprietor: Samsung Medison Co., Ltd., Kangwon-do 250-875 (KR)
(72) Inventor: Shin, Dong Kuk, 135-851, Seoul (KR); Jeong, Mok Kun, 137-768, Seoul (KR); Kim, Hye Jung, 135-851, Seoul (KR)
(74) Representative: Schmid, Wolfgang

(56) References cited:
- EP-A1- 0 920 833
- US-A- 5 474 070

## Description

### TECHNICAL FIELD

The present disclosure generally relates to ultrasound systems, and more particularly to providing stress information in an ultrasound system.

### BACKGROUND

Recently, an ultrasound system has been extensively used in the medical field due to its non-invasive and non-destructive nature. Modem high-performance ultrasound imaging systems and techniques are commonly used to produce two-dimensional ultrasound images and three-dimensional ultrasound images of internal features of patients.

Generally, the ultrasound image is displayed in a Brightness mode (B-mode) by using reflectivity caused by an acoustic impedance difference between the tissues of a target object. However, if the reflectivity of the target object is hardly different from those of the neighboring tissues such as tumor, cancer or the like, then it is not easy to discriminate the target object in the B-mode image. Further, an ultrasound elastic imaging technology has been developed to display an image of the target object by using mechanical characteristics of the target object. Such technology is very helpful for diagnosing lesions such as tumor or cancer, which is relatively stiffer than the neighboring tissues. When stress is uniformly applied to the target object, a variation of the tumor or cancer is typically smaller than those of the neighboring tissues. The elasticity of a tissue is measured by using ultrasound data obtained before and after applying stress to the target object.

A compression plate mounted on an ultrasound probe may be used to apply the stress to the target object. A user may press the compression plate on the target object to thereby apply the stress to the target object. In such a case, strain data cannot be obtained under the same stress since every user applies different stress. Thus, the video quality of an elastic image may be changed depending on the users.

Regarding the prior art in this field, reference is made to EP 0 920 833 A1 and US 5,474,070.

### SUMMARY

Embodiments for providing stress information in an ultrasound system are disclosed herein. In one embodiment by way of non-limiting example, an ultrasound system according to claim 1 is disclosed.

In another embodiment, there is provided a method of providing stress information according to claim 6.

In yet another embodiment, there is provided a computer readable medium according to claim 11.

The Summary is provided to introduce a selection of concepts in a simplified form that are further described below in the Detailed Description. This Summary is not intended to identify key or essential features of the claimed subject matter, nor is it intended to be used in determining the scope of the claimed subject matter.

### BRIEF DESCRIPTION OF THE DRAWINGS

- Figure 1: is a block diagram showing an ultrasound system in accordance with a first embodiment.
- Figure 2: is a block diagram showing an ultrasound data acquisition unit in accordance with the first embodiment.
- Figure 3: is a block diagram showing a processing unit in accordance with the first embodiment.
- Figure 4: is a schematic diagram showing an example of frames, elastic image frames, scan-lines and stress application periods.
- Figures 5 to 8: are schematic diagrams showing an example of stress information.
- Figure 9: is a block diagram showing an ultrasound system in accordance with a second embodiment.
- Figure 10: is a block diagram showing a processing unit in accordance with the second embodiment.

### DETAILED DESCRIPTION

A detailed description may be provided with reference to the accompanying drawings. One of ordinary skill in the art may realize that the following description is illustrative only and is not in any way limiting. Other embodiments of the present invention may readily suggest themselves to such skilled persons having the benefit of this disclosure.

### FIRST EMBODIMENT

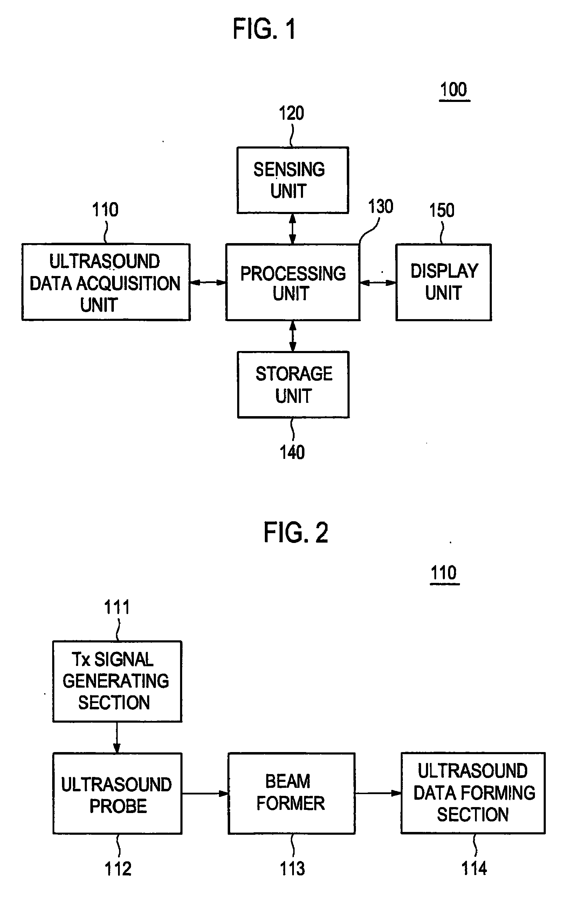

Referring to Figure 1, an ultrasound system 100 in accordance with an illustrative embodiment is shown. As depicted therein, the ultrasound system 100 may include an ultrasound data acquisition unit 110. The ultrasound data acquisition unit 110 may be configured to transmit/receive ultrasound signals to/from a target object to thereby acquire ultrasound data.

Referring to Figure 2, the ultrasound data acquisition unit 110 may include a transmit (Tx) signal generating section 111. The Tx signal generating section 111 may be configured to generate first Tx signals while applying stress to the target object ("first time duration"). The Tx signal generating section 111 may be further configured to generate second Tx signals while releasing the stress applied to the target object ("second time duration").

The ultrasound data acquisition unit 110 may further include an ultrasound probe 112 containing a plurality of elements for reciprocally converting between ultrasound signals and electrical signals. The ultrasound probe 112 may deliver the stress provided from an external to the target object. The ultrasound probe 112 may be configured to transmit ultrasound signals into the target object in response to the first Tx signals. The ultrasound probe 112 may be further configured to receive echo signals reflected from the target object (which received the first Tx signals) to thereby output first received signals. The ultrasound probe 112 may be also configured to transmit ultrasound signals into the target object in response to the second Tx signals. Moreover, the ultrasound probe 112 may be configured to the receive echo signals reflected from the target object (which received the second Tx signals) to thereby output second received signals.

The ultrasound data acquisition unit 110 may further include a beam former 113. The beam former 113 may be configured to convert the first received signals into first digital signals. The beam former 113 may be additionally configured to apply delays to the first digital signals in consideration of distance between the elements and focal points to thereby output first digital receive-focused signals. The beam former 113 may be further configured to convert the second received signals into second digital signals. The beam former 113 may be further configured to apply delays to the second digital signals in consideration of distance between the elements and the focal points to thereby output second digital receive-focused signals.

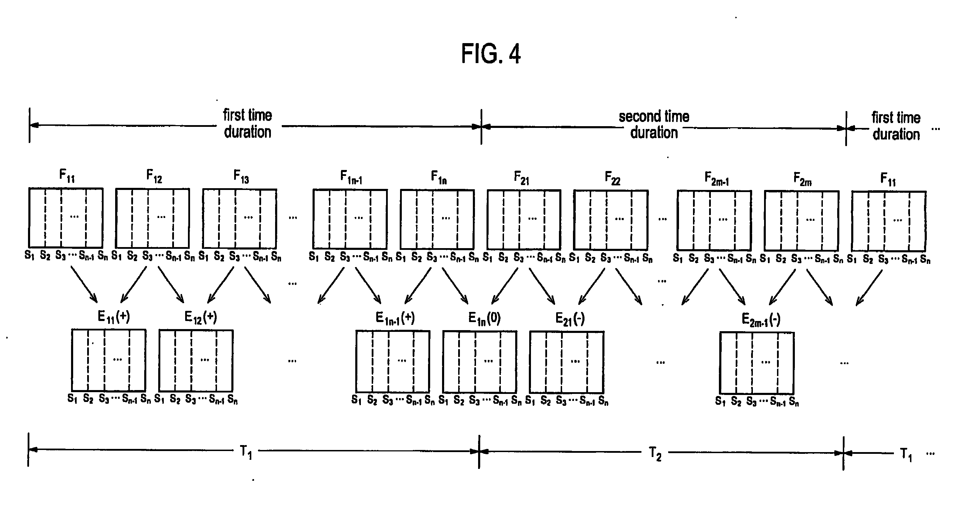

The ultrasound data acquisition unit 110 may further include an ultrasound data forming section 114. The ultrasound data forming section 114 may be configured to form first ultrasound data corresponding to each of first frames F₁₁ to F₁ₙ based on the first digital receive-focused signals, as shown in Figure 4. The ultrasound data forming section 114 may be further configured to form second ultrasound data corresponding to each of second frames F₂₁ to F₂ₘ based on the second digital receive-focused signals, as shown in Figure 4. The first and second ultrasound data may be radio frequency (RF) data or in-phase/quadrature (IQ) data.

Referring back to Figure 1, the ultrasound system 100 may further include a sensing unit 120. The sensing unit 120 may be configured to sense stress magnitude to thereby output the sensed stress magnitude signals. The sensing unit 120 may be attached to one side of a scan surface of the ultrasound probe 112.

The ultrasound system 100 may further include a processing unit 130 placed in communication with the ultrasound data acquisition unit 110 and the sensing unit 120. The processing unit 130 may be configured to form stress information based on the first and second ultrasound data provided from the ultrasound data acquisition unit 110 and the sensed stress magnitude signals provided from the sensing unit 120. The stress information may include stress magnitude information, stress application period information and ultrasound probe gradient information. Thus, a user may appropriately apply the stress to the target object by referring to the stress information displayed on a display unit 150. The processing unit 130 may be further configured to form an elastic image based on the first and second ultrasound data.

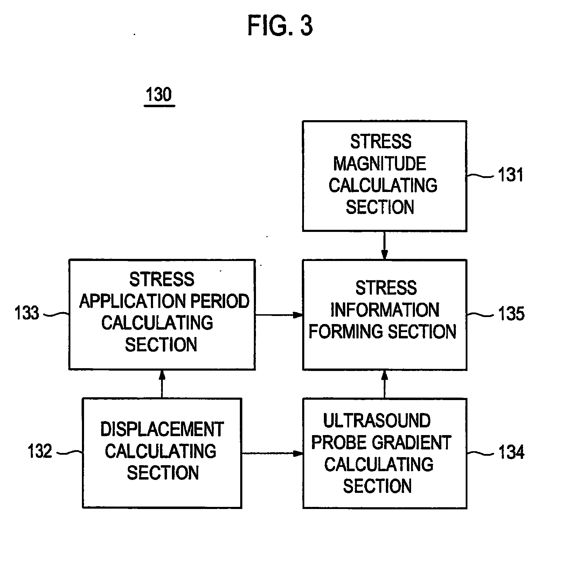

Referring to Figure 3, the processing unit 130 may include a stress magnitude calculating section 131, a displacement calculating section 132, a stress application period calculating section 133, an ultrasound probe gradient calculating section 134 and a stress information forming section 135.

The stress magnitude calculating section 131 may be configured to calculate stress magnitudes based on the sensed stress magnitude signals provided from the sensing unit 120. The methods of calculating the stress magnitude based on the sensed stress magnitude signals are well known in the art. Thus, they have not been described in detail so as not to unnecessarily obscure the present invention.

The displacement calculating section 132 may be configured to calculate displacements based on the first and second ultrasound data between the neighboring frames. The displacement may be calculated by using an auto-correlation method or a cross-correlation method. Referring to Figure 4, in one embodiment, the displacement calculating section 132 may be configured to calculate displacements corresponding to each of scan-lines S₁ to Sₙ of an elastic image frame E₁₁ based on the ultrasound data between the neighboring frames F₁₁ and F₁₂, as shown in Figure 4. Similarly, the displacement calculating section 132 may be further configured to calculate displacements corresponding to each of scan-lines S₁ to Sₙ of elastic image frames E₁₂, ···, E₁ₙ₋₁, E₁ₙ, E₂₁, ···, E₂ₘ₋₁ based on the ultrasound data between the neighboring frames F₁₂, F₁₃, ··· F₂ₘ₋₁ and F₂ₘ. In one embodiment, by way of non-limiting example, the displacements at the first time duration may have a positive sign, while the displacements at the second time duration may have a negative sign.

The stress application period calculating section 133 may be configured to calculate stress application periods based on the displacements provided from the displacement calculating section 132. In one embodiment, the stress application period calculating section 133 may be configured to calculate first time durations T₁ based on the displacements having the positive sign, and calculate second time durations T₂ based on the displacements having the negative sign, as shown in Figure 4.

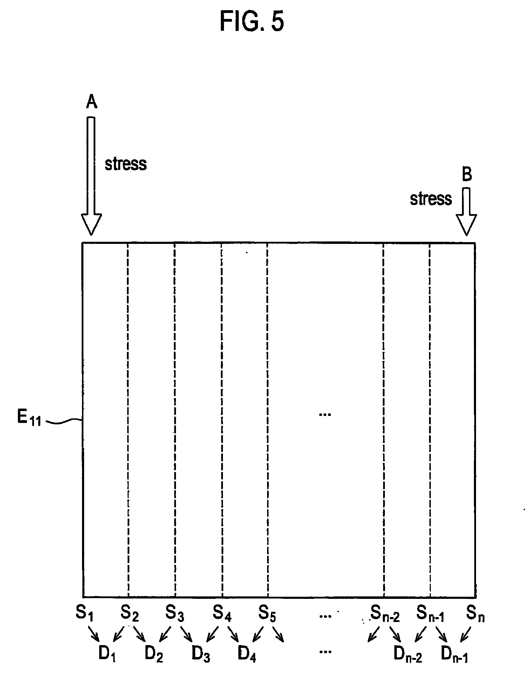

The magnitude of the stress, which is applied to the target object (not shown), may be changed along the scan-lines S₁ to Sₙ. As shown in Figure 5, the magnitude of the stress of the left side A may be larger than that of the right side B. The ultrasound probe gradient calculating section 134 may be configured to calculate ultrasound probe gradients of the ultrasound probe 112 based on the displacements provided from the displacement calculating section 132. In one embodiment, the ultrasound probe gradient calculating section 134 may be configured to calculate displacement differences D₁, D₂, ···, Dₙ₋₁ between the displacements corresponding to the neighboring scan-lines of the elastic image frame E₁₁ as shown in Figure 5. The ultrasound probe gradient calculating section 134 may be further configured to calculate an ultrasound probe gradient based on the calculated displacement differences. Similarly, the ultrasound probe gradient calculating section 134 may be further configured to calculate the displacement differences D₁, D₂, ···, Dₙ₋₁ between the displacements corresponding to the neighboring scan-lines of the elastic image frames E₁₂, ···, E₁ₙ₋₁, E₁ₙ, E₂₁, ···, E₂ₘ₋₁ and to calculate the ultrasound probe gradients based on the calculated displacement differences.

The stress information forming section 135 may be configured to form the stress information based on the stress magnitudes provided from the stress magnitude calculating section 131, the stress application periods provided from the stress application period calculating section 133 and the ultrasound probe gradients provided from the ultrasound probe gradient calculating section 134.

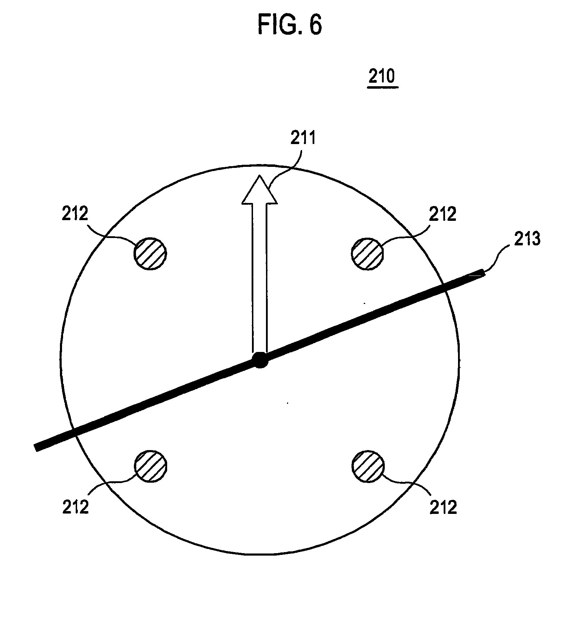

In one embodiment, the stress information forming section 135 may be configured to form stress magnitude information 211 representing the stress magnitude provided from the stress magnitude calculating section 131 as a size of a first symbol (e.g., arrow) based on the predetermined stress magnitude, as shown in Figure 6. The stress information forming section 135 may be further configured to form stress application period information 212 representing the stress application period provided from the stress application period calculating section 133 as a second symbol (e.g., circle) based on the predetermined stress application period. If the stress application period provided from the stress application period calculating section 133 is faster than the predetermined stress application period, then the stress information forming section 135 may be configured to form the stress application period information 212 representing a color of the second symbol as red (not shown). If the stress application period provided from the stress application period calculating section 133 is equal to the predetermined stress application period, then the stress information forming section 135 may be configured to form the stress application period information 212 representing the color of the second symbol as blue (not shown). If the stress application period provided from the stress application period calculating section 133 is slower than the predetermined stress application period, then the stress information forming section 135 may be configured to form the stress application period information 212 representing the color of the second symbol as black (not shown). The stress information forming section 135 may be further configured to form ultrasound probe gradient information 213 representing the ultrasound probe gradient provided from the ultrasound probe gradient calculating section 134 as a gradient of a third symbol. The stress information forming section 135 may be configured to form stress information 210 including the stress magnitude information 211, the stress application period information 212 and the ultrasound probe gradient information 213.

In other embodiment, the stress information forming section 135 may be configured to form stress magnitude information representing the stress magnitude provided from the stress magnitude as a size and color of the first symbol such as the arrow (not shown). If the stress magnitude provided from the stress magnitude calculating section 131 is larger than the predetermined stress magnitude, then the stress information forming section 135 may be configured to form the stress magnitude information representing the color of the first symbol as red (not shown). If the stress magnitude provided from the stress magnitude calculating section 131 is equal to the predetermined stress magnitude, then the stress information forming section 135 may be configured to form the stress magnitude information representing the color of the first symbol as blue (not shown). If the stress magnitude provided from the stress magnitude calculating section 131 is smaller than the predetermined stress magnitude, then the stress information forming section 135 may be configured to form the stress magnitude information representing the color of the first symbol as black (not shown). The stress information forming section 135 may form stress application period information, ultrasound probe gradient information and stress information, as described in the foregoing embodiment.

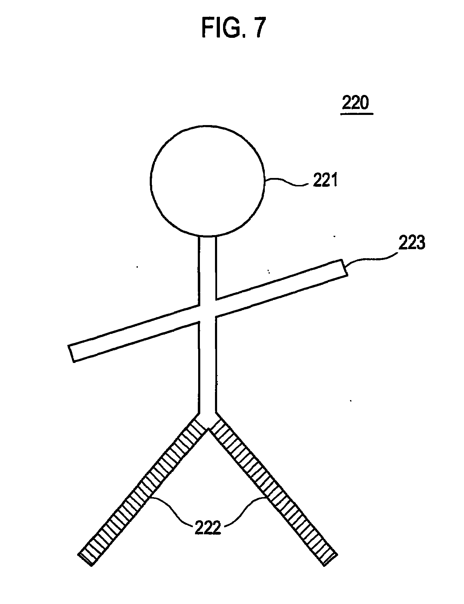

In another embodiment, the stress information forming section 135 may be configured to form stress magnitude information 221 representing the stress magnitude provided from the stress magnitude calculating section 131 as an expression of a character based on the predetermined stress magnitude as shown in Figure 7. If the stress magnitude provided from the stress magnitude calculating section 131 is equal to the predetermined stress magnitude, then the stress information forming section 135 may be configured to form the stress magnitude information 221 representing the stress magnitude provided from the stress magnitude calculating section 131 as a smile expression (not shown) of the character. If the stress magnitude provided from the stress magnitude calculating section 131 is not equal to the predetermined stress magnitude, then the stress information forming section 135 may be configured to form the stress magnitude information 221 representing the stress magnitude provided from the stress magnitude calculating section 131 as a wry expression (not shown) of the character. The stress information forming section 135 may be further configured to form stress application period information 222 representing the stress application period provided from the stress application period calculating section 133 as a motion and a color of legs of the character. If the stress application period provided from the stress application period calculating section 133 is faster than the predetermined stress application period, then the stress information forming section 135 may be configured to form the stress application period information 222 representing the color of the legs as red (not shown). If the stress application period provided from the stress application period calculating section 133 is equal to the predetermined stress application period, then the stress information forming section 135 may be configured to form the stress application period information 222 representing the color of the legs as blue (not shown). If the stress application period provided from the stress application period calculating section 133 is slower than the predetermined stress application period, then the stress information forming section 135 may be configured to form the stress application period information 222 representing the color of the legs as black (not shown). The stress information forming section 135 may be further configured to form ultrasound probe gradient information 223 representing the ultrasound probe gradient provided from the ultrasound probe gradient calculating section 134 as a gradient of arms of the character. The stress information forming section 135 may be configured to stress information 220 including the stress magnitude information 221, the stress application period information 222 and the ultrasound probe gradient information 223.

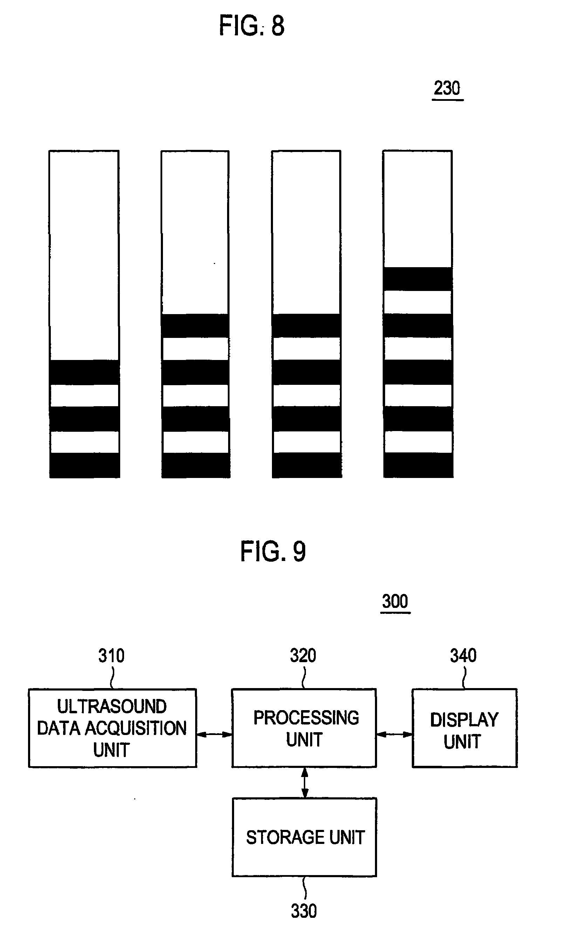

In yet another embodiment, the stress information forming section 135 may be configured to form stress magnitude information representing the stress magnitude provided from the stress magnitude calculating section 131 as a height of graph bar based on the predetermined stress magnitude as shown in Figure 8. The stress information forming section 135 may be further configured to form stress application period information representing the stress application period provided from the stress application period calculating section 133 as a color of the graph bar based on the predetermined stress application period as shown in Figure 8. If the stress application period provided from the stress application period calculating section 133 is faster than the predetermined stress application period, then the stress information forming section 135 may be configured to form the stress application period information representing the color of the graph bar as red (not shown). If the stress application period provided from the stress application period calculating section 133 is equal to the predetermined stress application period, then the stress information forming section 135 may be configured to form the stress application period information representing the color of the graph bar as blue (not shown). If the stress application period provided from the stress application period calculating section 133 is slower than the predetermined stress application period, then the stress information forming section 135 may be configured to form the stress application period information representing the color of the graph bar as black (not shown). The stress information forming section 135 may be further configured to form ultrasound probe gradient information representing the ultrasound probe gradient as a gradient of graph bar. The guide information forming section 135 may be further configured to form guide information 230 including the stress magnitude information, the stress application period information and the ultrasound probe gradient information.

While the stress magnitude, the stress application period and the ultrasound probe gradient are represented as the symbol and/or the color in foregoing embodiments, the stress magnitude, the stress application period and the ultrasound probe gradient are represented as at least one of a symbol, a graph, numerical value, text and colors.
Referring back to Figure 1, the ultrasound system 100 may further include a storage unit 140. The storage unit 140 may store the ultrasound data acquired from the ultrasound data acquisition unit 110. The storage unit 140 may further store the stress information formed from the processing unit 130.

The ultrasound system 100 may further include the display unit 150. The display unit 150 may display the stress information formed from the processing unit 130. The display unit 150 may further display the elastic image formed from the processing unit 130.

### SECOND EMBODIMENT

Referring to Figure 9, an ultrasound system 300 in accordance with a second embodiment is shown. As depicted therein, the ultrasound system 300 may include an ultrasound data acquisition unit 310. The ultrasound data acquisition unit 310 may be configured to transmit/receive ultrasound signals to/from a target object to thereby acquire ultrasound data. The ultrasound data acquisition unit 310 in the second embodiment is similar to the ultrasound data acquisition unit 110 in the first embodiment shown in Figure 1. Thus, the ultrasound data acquisition unit 310 in the present embodiment has not been described in detail.

The ultrasound system 300 may further include processing unit 320. The processing unit 320 may be configured to form stress information based on the ultrasound data provided from the ultrasound data acquisition unit 310. The stress information may include stress magnitude information, stress application period information and ultrasound probe gradient information. Thus, a user may appropriately apply the stress to the target object by referring to the stress information. The processing unit 320 may be further configured to form an elastic image based on the ultrasound data.

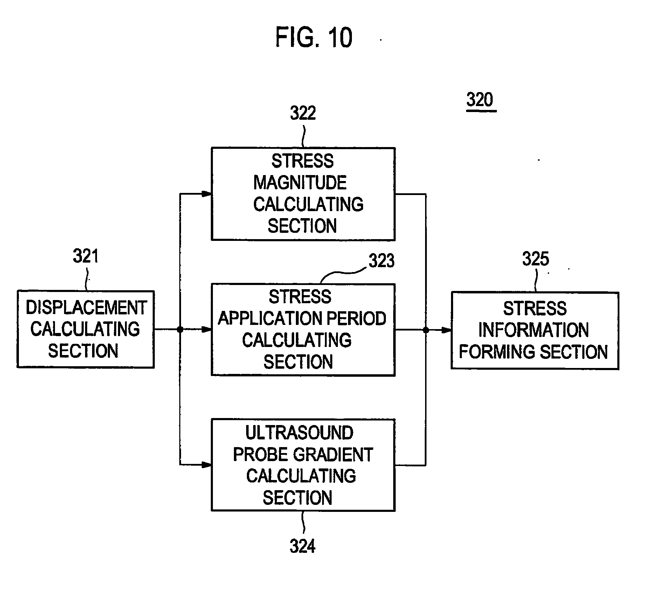

Figure 10 is a block diagram showing an illustrative embodiment of the processing unit 320. Referring to Figure 10, the processing unit 320 may include a displacement calculating section 321, a stress magnitude calculating section 322, a stress application period calculating section 323, an ultrasound probe gradient calculating section 324 and a stress information forming section 325.

The displacement calculating section 321 may be configured to calculate displacements based on the ultrasound data between the neighboring frames. The displacement may be calculated by using an auto-correlation method or a cross-correlation method. The displacement calculating section 321 in the second embodiment is similar to the displacement calculating section 132 shown in Figure 3. Thus, it has not been described in detail.

The stress magnitude calculating section 322 may be configured to calculate stress magnitudes corresponding to each of scan-lines S₁ to Sₙ of elastic image frames E₁₁, E₁₂, ···, E₁ₙ₋₁, E₁ₙ, E₂₁, ···, E₂ₘ₋₁ based on the displacements provided from the displacement calculating section 321, as shown in Figure 4. The methods of calculating the stress magnitude based on the displacements are well known in the art. Thus, they have not been described in detail so as not to unnecessarily obscure the present invention.

The stress application period calculating section 323 may be configured to calculate stress application periods based on the displacements provided from the displacement calculating section 321. The stress application period calculating section 323 in the second embodiment is similar to the stress application period 133 in the first embodiment shown in Figure 3. Thus, it has not been described in detail.

The ultrasound probe gradient calculating section 324 may be configured to calculate ultrasound probe gradients of the ultrasound probe 112 based on the displacements provided from the displacement calculating section 321. The ultrasound probe gradient calculating section 324 in the second embodiment is similar to the ultrasound probe gradient calculating section 134 in the first embodiment shown in Figure 3. Thus, it has not been described in detail.

The stress information forming section 325 may be configured to form the stress information including the stress magnitude information, the stress application period information and the ultrasound probe gradient information based on the stress magnitudes, the stress application periods and the ultrasound probe. The stress information forming section 325 in the second embodiment is similar to the stress information forming section 135 in the first embodiment shown in Figure 3. Thus, it has not been described in detail.

Referring back to Figure 9, the ultrasound system 300 may further include a storage unit 330. The storage unit 330 may store the ultrasound data acquired from the ultrasound data acquisition unit 310. The storage unit 330 may further store the stress information formed from the processing unit 320.

The ultrasound system 300 may further include a display unit 340. The display unit 340 may display the stress information formed from the processing unit 320. The display unit 340 may further display the elastic image formed from the processing unit 320.

## Claims

1. An ultrasound system (100, 300), comprising:
an ultrasound data acquisition unit (110, 310) configured to transmit/receive ultrasound signals to/from a target object through an ultrasound probe (112) while applying a stress to the target object to thereby output first ultrasound data and to transmit/receive ultrasound signals to/from the target object through the ultrasound probe (112) while releasing the stress applied to the target object to thereby output second ultrasound data;
a processing unit (130, 320) in communication with the ultrasound data acquisition unit (110, 310) and being configured to calculate stress magnitudes of the stress, stress application periods representing time durations of the stress application and release, and ultrasound probe gradients representing gradients of the ultrasound probe (112) based on displacement differences between displacements corresponding to neighboring scan-lines and form stress information based on the the stress magnitudes, the stress application periods and the ultrasound probe gradients, wherein the stress information includes stress magnitude information corresponding to the stress magnitudes, stress application period information corresponding to the stress application periods and ultrasound probe gradient information corresponding to the ultrasound probe gradient; and
a display unit (150, 340) configured to display the stress information.

2. The ultrasound system (100, 300) of Claim 1, wherein the stress information includes at least one of a symbol, a graph, numerical value, text and colors.

3. The ultrasound system (100) of Claim 1, further comprising a sensing unit (120) in communication with the processing unit (130), the sensing unit (120) being configured to sense the stress magnitudes of the stress applied to the target object to thereby transit the sensed stress magnitude signals to the processing unit (130).

4. The ultrasound system (100) of Claim 3, wherein the processing unit (130) comprises:
a stress magnitude calculating section (131) configured to calculate the stress magnitudes based on the sensed stress magnitude signals;
a displacement calculating section (132) configured to calculate the displacements based on the first and second ultrasound data;
a stress application period calculating section (133) configured to calculate the stress application periods based on the calculated displacements;
an ultrasound probe gradient calculating section (134) configured to calculate the ultrasound probe gradients based on the calculated displacements; and
a stress information forming section (135) configured to form the stress magnitude information based on the calculated stress magnitudes, form the stress application period information based on the calculated stress application periods and form the ultrasound probe gradient information based on the calculated ultrasound probe gradients, the stress information forming section being further configured to form the stress information including the stress magnitude information, the stress application period information and the ultrasound probe gradient information.

5. The ultrasound system (300) of Claim 1, wherein the processing unit (320) comprises:
a displacement calculating section (321) configured to calculate the displacements based on the first and second ultrasound data;
a stress magnitude calculating section (322) configured to calculate the stress magnitudes based on the calculated displacements;
a stress application period calculating section (323) configured to calculate the stress application periods based on the calculated displacements;
an ultrasound probe gradient calculating section (324) configured to calculate the ultrasound probe gradients based on the calculated displacements; and
a stress information forming section (325) configured to form the stress magnitude information based on the calculated stress magnitudes, form the stress application period information based on the calculated stress application periods and form the ultrasound probe gradient information based on the calculated ultrasound probe gradients, the stress information forming section being further configured to form the stress information including the stress magnitude information, the stress application period information and the ultrasound probe gradient information.

6. A method of providing stress information, comprising:
a) transmitting/receiving ultrasound signals to/from a target object through an ultrasound probe while applying a stress to the target object to thereby output first ultrasound data;
b) transmitting/receiving ultrasound signals to/from the target object through the ultrasound probe white releasing the stress applied to the target object to thereby output second ultrasound data;
c) calculating stress magnitudes of the stress, stress application periods representing time durations of the stress application and release, and ultrasound probe gradients represent-ing gradients of the ultrasound probe based on displacement differences between displacements corresponding to neighboring scan-liner
d) forming stress information based on the stress magnitudes, the stress application periods and the ultrasound probe gradients, wherein the stress information includes stress magnitude information corresponding to the stress magnitudes, stress application period information corresponding to the stress application periods and ultrasound probe gradient information corresponding to the ultrasound probe gradient; and
e) displaying the stress information on a display unit.

7. The method of Claim 6, wherein the stress information includes at least one of a symbol, a graph, numerical value, text, colors.

8. The method of Claim 6, further comprising the step of sensing the stress magnitudes to thereby output the sensed stress magnitude signals.

9. The method of Claim 8, wherein the step c) comprises:
calculating the stress magnitudes based on the sensed stress magnitude signals;
calculating the displacements based on the first and second ultrasound data;
calculating the stress application periods based on the calculated displacements; and
calculating the ultrasound probe gradients based on the calculated displacements, and
the step d) comprises:
forming the stress magnitude information based on the calculated stress magnitudes;
forming the stress application period information based on the calculated stress application periods;
forming the ultrasound probe gradient information based on the calculated ultrasound probe gradients; and
forming the stress information including the stress magnitude information, the stress application period information and the ultrasound probe gradient information.

10. The method of Claim 6, wherein the step c) comprises:
calculating the displacements based on the first and second ultrasound data; and
calculating the stress magnitudes based on the calculated displacements, and
the step d) comprises:
forming the stress magnitude information based on the calculated stress magnitudes;
forming the stress application period information based on the calculated stress application periods;
forming the ultrasound probe gradient information based on the calculated ultrasound probe gradients; and
forming the stress information including the stress magnitude information, the stress application period information and the ultrasound probe gradient information.

11. A computer readable medium comprising computer executable instructions configured to perform following acts:
a) transmitting/receiving ultrasound signals to/from a target object while applying a stress to the target object to thereby output first ultrasound data;
b) transmitting/receiving ultrasound signals to/from the target object while releasing the stress applied to the target object to thereby output second ultrasound data;
c) calculating stress magnitudes of the stress, stress application periods representing time durations of the stress application and release, and ultrasound probe gradients representing gradients of the ultrasound probe based on displacement differences between displacements comprising to neighboring scan-lines;
d) forming stress information based on the stress magnitudes, the stress application periods and the ultrasound probe gradients, wherein the stress information includes stress magnitude information corresponding to the stress magnitudes, stress application period information corresponding to the stress application periods and ultrasound probe gradient information corresponding to the ultrasound probe gradient; and
e) displaying the stress information.

12. The computer readable medium of claim 11, wherein the stress information includes at least one of a symbol a graph, numerical value, text, colors.

13. The computer readable medium of claim 11, further comprising computer executable instructions configured to preform the act of sensing the stress magnitudes to thereby output the sensed stress magnitude signals.

14. The computer readable medium of claim 13, wherein the act c) comprises:
calculating the stress magnitudes based on the sensed stress magnitude signals; and
calculating the displacements based on the first and second ultrasound data, and
the act d) comprises:
forming the stress magnitude information based on the calculated stress magnitudes;
forming the stress application period information based on the calculated stress application periods;
forming the ultrasound probe gradient information based on the calculated ultrasound probe gradients; and
forming the stress information including the stress magnitude information, the stress application period information and the ultrasound probe gradient information.

## Patentansprüche

1. Ultraschallsystem (100,300), welches Folgendes aufweist:
eine Ultraschalldaten-Erlangungseinheit (110,310), die dafür vorgesehen ist, Ultraschallsignale zu bzw. von einem Zielobjekt durch eine Ultraschallsonde (112) zu übertragen bzw. zu empfangen, während sie eine Spannung auf das Zielobjekt aufbringt_{;} um dadurch erste Ultraschalldaten auszugeben, und Ultraschallsignale zu bzw. von dem Zielobjekt durch die Ultraschallsonde (112) zu übertragen bzw. zu empfangen, während die auf das Zielobjekt aufgebrachte Spannung gelöst wird, um dadurch zweite Ultraschalldaten auszugeben;
eine Verarbeitungseinheit (130, 320), die in Verbindung mit der Ultraschalldaten-Erlangungseinheit (110,310), steht und dafür vorgesehen ist, Spannungsgrößen der Spannung, Spannungsaufbringungszeitabschnitte, die den Zeiten der Spannungsaufbringung und -lösung entsprechen, und Ultraschallsondengradienten, die Gradienten der Ultraschallsonde (112) repräsentieren, basierend auf Verschiebungsunterschieden zwischen mit benachbarten Scanlinien korrespondierenden Verschiebungen zu berechnen, und Spannungsinformationen basierend auf den Spannungsgrößen, den Spannungsaufbringungszeitabschnitten und den Ultraschallsondengradienten zu bilden, wobei die Spannungsinformationen den Spannungsgrößen entsprechende Spannungsgrößeninformationen, den Spannungsaufbringungszeitabschnitten entsprechende Spannungsaufbringungszeitabschnittsinformationen und den Ultraschallsondengradienten entsprechende Ultraschallsondengradienteninformationen beinhalten; und
eine Anzeigeneinheit (150,340), die dafür vorgesehen ist, die Spannungsinformationen anzuzeigen.

2. Ultraschallsystem (100,300) nach Anspruch 1, wobei die Spannungsinformationen wenigstens eines von einem Symbol, einem Diagramm, einem nummerischen Wert, einem Text und Farben beinhalten.

3. Ultraschallsystem (100) nach Anspruch 1, welches des Weiteren eine Abtasteinheit (120) aufweist, die in Verbindung mit der Verarbeitungseinheit (130) steht, wobei die Abtasteinheit (120) dafür vorgesehen ist, Spannungsgrößen der auf das Zielobjekt aufgebrachten Spannung abzutasten, um dadurch die abgetasteten Spannungsgrößensignale zu der Verarbeitungseinheit (130) zu übertragen.

4. Ultraschallsystem (100) nach Anspruch 3, wobei die Verarbeitungseinheit (130) Folgendes aufweist:
einen Spannungsgrößenberechnungsabschnitt (131), der dafür vorgesehen ist, die Spannungsgrößen basierend auf den abgetasteten Spannungsgrößensignalen zu berechnen;
einen Verschiebungsberechnungsabschnitt (132), welcher dafür vorgesehen ist, die Verschiebungen basierend auf den ersten und zweiten Ultraschalldaten zu berechnen;
einen Spannungsaufbringungszeitdauerberechnungsabschnitt (133), welcher dafür vorgesehen ist, die Zeitdauer bzw. Zeitabschnitte der Spannuhgsaufbringung basierend auf den berechneten Verschiebungen zu berechnen;
einen Ultraschallsondengradientenberechnungsabschnitt (134), welcher dafür vorgesehen ist, die Ultraschallsondengradienten basierend auf den berechneten Verschiebungen zu berechnen; und
einen Spannungsinformationsbildungsabschnitt (135), welcher dafür vorgesehen ist, die Spannungsgrößeninformationen basierend auf den berechneten Spannungsgräßen zu bilden, die Spannungsaufbringungszeitdauerinformationen basierend auf den berechneten Spannungsaufbringungszeitabschnitten zu bilden und die Ultraschallsondengradienteninformationen basierend auf den berechneten Ultraschallsondengradienten zu bilden, wobei der Spannungsinformationsbildungsabschnitt des Weiteren dafür vorgesehen ist, die Spannungsinformationen einschließlich der Spannungsgrößeninformationen, der Spannungsaufbringungszeitabschnittsinformationen und der Ultraschallsondengradienteninformationen zu bilden.

5. Ultraschallsystem (300) nach Anspruch 1, wobei die Verarbeitungseinheit (320) Folgendes aufweist:
einen Verschiebungsberechnungsabschnitt (321), der dafür vorgesehen ist, die Verschiebungen basierend auf den ersten und zweiten Ultraschalldaten zu berechnen;
einen Spannungsgrößenberechnungsabschnitt (322), welcher dafür vorgesehen ist, die Spannungsgrößen basierend auf den berechneten Verschiebungen zu berechnen;
einen Spannungsaufbringungszeitdauerberechnungsabschnitt (323), welcher dafür vorgesehen ist, die Zeitdauer bzw. Zeitabschnitte der Spannungsaufbringung basierend auf den berechneten Verschiebungen zu berechnen;
einen Ultraschallsondengradientenberechnungsabschnitt (324), welcher dafür vorgesehen ist, die Ultraschallsondengradienten basierend auf den berechneten Verschiebungen zu berechnen; und
einen Spannungsinformationsbildungsabschnitt (325), welcher dafür vorgesehen ist, die Spannungsgrößeninformationen basierend auf den berechneten Spannungsgrößen zu bilden, die Spannungsaufbringungszeitdauerinformationen basierend auf den berechneten Spannungsaufbringungszeitabschnitten zu bilden und die Ultraschallsondengradienteninformationen basierend auf den berechneten Ultraschallsondengradienten zu bilden, wobei der Spannungsinformationsbildungsabschnitt des Weiteren dafür vorgesehen ist, die Spannungsinformationen einschließlich der Spannungsgrößeninformationen, der Spannungsaufbringungszeitabschnittsinformationen und der Ultraschallsondengradienteninformationen zu bilden.

6. Verfahren zum Erzeugen von Spannungsinformationen, welches Folgendes aufweist:
a) Übertragen bzw. Empfangen von Ultraschallsignalen zu bzw. von einem Zielobjekt durch eine Ultraschallsonde, während eine Spannung auf das Zielobjekt aufgebracht wird, um dadurch erste Ultraschalldaten auszugeben;
b) Übertragen bzw. Empfangen von Ultraschallsignalen zu bzw. von dem Zielobjet durch die Ultraschallsonde, während die auf das Zielobjekt aufgebrachte Spannung gelöst wird, um dadurch zweite Ultraschalldaten auszugeben;
c) Berechnen von Spannungsgrößen der Spannung, Spannungsaufbringungszeitabschnitte, die den Zeiten der Spannungsaufbringung und -lösung entsprechen, und Ultraschallsondengradienten, die Gradienten der Ultraschallsonde repräsentieren, basierend auf Verschiebungsunterschieden zwischen mit benachbarten Scanlinien korrespondierenden Verschiebungen zu berechnen;
d) Bilden von Spannungsinformationen basierend auf den Spannungsgrößen, den Spannungsaufbringungszeitabschnitten und den Ultraschallsondengradienten, wobei die Spannungsinformationen den Spannungsgrößen entsprechende Spannungsgrößeninformationen, den Spannungsaufbringungszeitabschnitten entsprechende Spannungsaufbringungszeitabschnittsinformationen und den Ultraschallsondengradienten entsprechende Ultraschallsondengradienteninformationen beinhalten; und
e) Anzeigen der Spannungsinformationen auf einer Anzeigeeinheit.

7. Verfahren nach Anspruch 6, wobei die Spannungsinformationen wenigstens eines von einem Symbol, einem Diagramm, einem nummerischen Wert, einem Text und Farben beinhalter.

8. Verfahren nach Anspruch 6, welches des Weiteren den Schritt des Abtastens der Spannungsgrößen aufweist, um dadurch die abgetasteten Spannungsgrößensignale auszugeben.

9. Verfahren nach Anspruch 8, wobei der Schritt c) Folgendes aufweist:
Berechnen der Spannungsgrößen basierend auf den abgetasteten Spannungsgrößensignalen;
Berechnen der Verschiebungen basierend auf den ersten und zweiten Ultraschalldaten;
Berechnen der Spannungsaufbringungszeitabschnitten basierend auf den berechneten Verschiebungen; und
Berechnen der Ultraschallsondengradienten basierend auf den berechneten Verschiebungen, und wobei der Schritt d) Folgendes aufweist:
Bilden der Spannungsgrößeninformationen basierend auf den berechneten Spannungsgrößen;
Bilden der Spannungsaufbringungszeitdauerinformationen basierend auf den berechneten Spannungsaufbringungszeitabschnitten;
Bilden der Ultraschallsondengradienteninformationen basierend auf den berechneten Ultraschallsondengradienten; und
Bilden der Spannungsinformationen einschließlich der Spannungsgrößeninformationen der Spannungsaufbringungszeitabschnittsinformationen und der Ultraschallsondengradienteninformationen.

10. Verfahren nach Anspruch 6, wobei der Schritt c) Folgendes aufweist:
Berechnen der Verschiebungen basierend auf den ersten und zweiten Ultraschalldaten; und
Berechnen der Spannungsgrößen basierend auf den berechneten Verschiebungen, und wobei der Schritt d) Folgendes aufweist:
Bilden der Spannungsgrößeninförmationen basierend auf den berechneten Spannungsgrößen;
Bilden der Spannungsaufbringungszeitdauerinformationen basierend auf den berechneten Spannungsaufbringungszeitdauern;
Bilden der Ultraschallsondengradienteninformationen basierend auf den berechneten Ultraschall sondengradienten; und
Bilden der Spannungsinformationen einschließlich der Spannungsgrößeninformationen, der Spannungsaufbringungszeitabschnittsinformationen und der Ultraschallsondengradienteninformationen.

11. Computerlesbares Medium mit von einem Computer ausführbaren Befehlen, welches dafür vorgesehen ist, die folgenden Vorgänge auszuführen:
a) Übertragen bzw. Empfangen von Ultraschallsignalen zu bzw. von einem Zielobjekt, während eine Spannung auf das Zielobjekt aufgebracht wird, um dadurch erste Ultraschalldaten auszugeben;
b) Übertragen bzw. Empfangen von Ultraschallsignalen zu bzw. von dem Zielobjekt, während die auf das Zielobjekt aufgebrachte Spannung gelöst wird, um dadurch zweite Ultraschalldaten auszugeben;
c) Berechnen von Spannungsgrößen der Spannung, Spannungsaufbringungszeitabschnitte, die den Zeiten der Spannungsaufbringung und -lösung entsprechen, und Ulfraschallsondengradienten, die Gradienten der Ultraschallsonde repräsentieren, basierend auf Verschiebungsunterschieden zwischen mit benachbarten Scanlinien korrespondierenden Verschiebungen zu berechnen;
d) Bilden von Spannungsinformationen basierend auf den Spannungsgrößen, den Spannungsaufbringungszeitabschnitten und den Ultraschallsondengradienten, wobei die Spannungsinformationen den Spannungsgrößen entsprechende Spannungsgrößeninformationen, den Spannungsaufbringungszeitabschnitten entsprechende Spannungsaufbringungszeitabschnittsinformationen und den Ultraschallsondengradienten entsprechende Ultraschallsondengradienteninformationen beinhalten; und
e) Anzeigen der Spannungsinformationen.

12. Computerlesbares Medium nach Anspruch 11, wobei die Spannungsinformationen wenigstens eines von einem Symbol, einem Diagramm, einem nummerischen Wert, einem Text und Farben beinhalten.

13. Computerlesbares Medium nach Anspruch 11, welches des Weiteren von einem Computer ausführbare Befehle aufweist, die dafür vorgesehen sind, den Vorgang des Abtastens der Spannungsgrößen auszuführen, um dadurch die abgetasteten Spannungsgrößensignale auszugeben.

14. Computerlesbares Medium nach Anspruch 13, wobei der Vorgang c) Folgendes aufweist:
Berechnen der Spannungsgrößen basierend auf den abgetasteten Spannungsgrößensignalen; und
Berechnen der Verschiebungen basierend auf den ersten und zweiten Ultraschalldaten, und wobei der Vorgang d) Folgendes aufweist:
Bilden der Spannungsgrößeninformationen basierend auf den berechneten Spannungsgrößen;
Bilden der Spannungsaufbringungszeitdauerinformationen basierend auf den berechneten Spannungsaufbringungszeitabschnitten;
Bilden der Ultraschallsondengradienteninformationen basierend auf den berechneten Ultraschallsondengradienten; und
Bildender Spannungsinformationen einschließlich der Spannungsgrößeninformationen der Spannungsaufbringungszeitabschnittsinformationen und der Ultraschallsondengradienteninformationen.

## Revendications

1. Système ultrasonique (100, 300) comportant :
une unité d'acquisition de données ultrasoniques. (110, 310) agencée pour transmettre/ recevoir des signaux ultrasoniques vers/de la part d'un objet cible au moyen d'une sonde ultrasonique (112) pendant l'application d'une contrainte sur l'objet cible pour obtenir des premières données ultrasoniques et pour transmettre/recevoir des signaux ultrasoniques de l'objet cible au moyen de la sonde ultrasonique (112) pendant le relâchement de la contrainte appliquée sur l'objet cible pour obtenir des secondes données ultrasoniques,
une unité de calcul (130, 320) en liaison avec l'unité d'acquisition de données ultrasoniques (110, 310), agencée pour calculer des amplitudes de contraintes, des périodes d'application des contraintes représentant des durées de l'application de contraintes et du relâchement, et des pentes de sonde ultrasonique représentant des pentes de la sonde ultrasonique (112) basées sur les différences de déplacements entre les déplacements correspondant aux lignes de balayage voisines, et pour déterminer des informations de contraintes basées sur les amplitudes de contraintes, les périodes d'application des contraintes et les pentes de sonde ultrasonique, dans lequel, les informations de contrainte englobent les informations d'amplitude des contraintes correspondant aux amplitudes des contraintes, les informations relatives aux périodes d'application des contraintes, correspondant aux périodes d'application de contraintes et les informations relatives à la pente de la sonde correspondant à la pente de la sonde ultrasonique, et
une unité d'affichage (150, 340) agencée pour afficher l'information relative à la contrainte.

2. Système ultrasonique (100, 300) selon la revendication 1, dans lequel l'information relative à la contrainte inclut au moins un symbole, un graphique, une valeur numérique, un texte et des couleurs.

3. Système ultrasonique (100) selon la revendication 1, comportant en outre une unité de détection (120) couplée à l'unité de calcul (130), l'unité de détection (120) étant agencée pour détecter les amplitudes de contraintes des contraintes appliquées sur l'objet cible afin de transmettre ainsi des signaux correspondant à l'amplitude de la contrainte appliquée, vers l'unité de calcul (130).

4. Système ultrasonique (100) selon la revendication 3, dans lequel l'unité de calcul (130) comprend :
une section de calcul de l'amplitude de la contrainte (131) agencée pour calculer l'amplitude de la contrainte basée sur les signaux de détection de l'amplitude de la contrainte ;
une section de calcul du déplacement (132) agencée pour calculer les déplacements basés sur les premières et les secondes données ultrasoniques ;
une section de calcul de la période de l'application de la contrainte (133) agencée pour calculer les périodes d'application de contraintes basées sur les déplacements calculés;
une section de calcul de la pente de la sonde ultrasonique (134) agencée pour calculer les pentes de la sonde ultrasonique basées sur les déplacements calculés; et
une section d'information relative à la contrainte (135) agencée pour déterminer l'information relative à l'amplitude de la contrainte basée sur les amplitudes des contraintes calculées, pour déterminer l'information relative à la période d'application de la contrainte basée sur le calcul des périodes d'application de contraintes et pour constituer l'information relative à la pente de la sonde ultrasonique basée sur le calcul des pentes de la sonde ultrasonique, la section d'information relative à la contrainte étant en outre agencée pour déterminer l'information relative à la contrainte incluant l'information relative à l'amplitude de la contrainte, l'information relative à la période d'application de la contrainte et l'information relative à la pente de la sonde ultrasonique.

5. Système ultrasonique (300) selon la revendication 1, dans lequel l'unité de calcul (320) comprend :
une section de calcul du déplacement (321) agencée pour calculer les déplacements basés sur les premières et les secondes données ultrasoniques;
une section de calcul des amplitudes des contraintes (322), agencée pour calculer les amplitudes des contraintes basées sur les déplacements calculés;
une section de calcul de la période d'application de la contrainte (323) agencée pour calculer les périodes d'application de contraintes, basées sur les déplacements calculés;
une section de calcul de la pente de la sonde ultrasonique (324) agencée pour calculer les pentes de la sonde ultrasonique basées sur les déplacements calculés, et
une section relative à l'information de contrainte (325), pour déterminer l'information relative à l'amplitude de la contrainte basée sur les amplitudes de contraintes calculées, pour déterminer l'information relative à la période de contrainte basée sur les périodes d'application de contraintes calculées et pour déterminer l'information relative à la pente de la sonde ultrasonique basée sur les pentes calculées de la sonde ultrasonique, la section de détermination de l'information relative à la contrainte étant en outre agencée pour déterminer l'information relative à la contrainte incluant l'information relative à l'amplitude de la contrainte, l'information relative à la période d'application de la contrainte et l'information relative à la pente de la sonde ultrasonique.

6. Procédé pour déterminer l'information relative à la contrainte, comportant :
a) la transmission/ réception de signaux ultrasonique vers/de la part d'un objet cible au moyen d'une sonde ultrasonique pendant l'application d'une contrainte sur l'objet cible pour obtenir des premières données ultrasoniques ;
b) la transmission/ réception de signaux ultrasoniques vers/de la part d'un objet cible au moyen d'une sonde ultrasonique pendant le relâchement de la contrainte appliquée sur l'objet cible pour obtenir des secondes données ultrasoniques,
c) le calcul des amplitudes de contraintes, des périodes d'application des contraintes représentant des durées d'application de contraintes et du relâchement, et des pentes de la sonde ultrasonique représentant les pentes de la sonde ultrasonique basées sur les différences de déplacements entre les déplacements correspondant aux lignes de balayage voisines,
d) la détermination des informations relatives aux contraintes basées sur les amplitudes de contraintes, les périodes d'application des contraintes et les pentes de sonde ultrasonique, dans lequel, les informations relative aux contraintes incluent les informations d'amplitude des contraintes correspondant aux amplitudes des contraintes, les informations d'application des contraintes, correspondant aux périodes d'application des contraintes et les informations de pente de la sonde correspondant à la pente de la sonde ultrasonique ; et
e) l'affichage de l'information relative à la contrainte sur l'unité d'affichage.

7. Procédé selon la revendication 6, dans lequel l'information relative à la contrainte comprend en outre au moins un symbole, un graphique, une valeur numérique, un texte et des couleurs.

8. Procédé selon la revendication 6, comprenant en outre une phase de détection des amplitudes de contraintes afin de déterminer des signaux correspondant à l'amplitude de la contrainte.

9. Procédé selon la revendication 8, dans lequel, l'étape c) comprend :
le calcul des amplitudes de contraintes basées sur les signaux captés relatifs à l'amplitude des contraintes ;
le calcul des déplacements basés sur les premières et les secondes données ultrasoniques;
le calcul des périodes d'applications de contraintes basées sur les déplacements calculés ; et
le calcul des pentes de la sonde ultrasonique basées sur les déplacements calculés, et
l'étape d) comprend :
la détermination de l'information relative à l'amplitude de la contrainte basée sur les amplitudes calculées des contraintes ;
la détermination de l'information relative à la période d'application de la contrainte basée sur les périodes calculées d'application des contraintes ;
la détermination de l'information relative à la pente de la sonde ultrasonique basée sur les pentes calculées de la sonde ultrasonique ; et
la détermination de l'information relative à la contrainte incluant l'information relative à l'amplitude dé la contrainte, l'information relative à la période d'application de la contrainte et l'information relative à la pente de la sonde ultrasonique.

10. Procédé selon la revendication 6, dan lequel, l'étap c) comprend :
le calcul des déplacements basés sur les premières et les secondes données ultrasoniques ; et
le calcul des amplitudes de contraintes basées sur les déplacements calculés; et
l'étape d) comprend :
la détermination de l'information relative à l'amplitude de la contrainte basée sur les amplitudes calculées des contraintes ;
la détermination de l'information relative à la période d'application de la contrainte basée sur les périodes calculées d'application des contraintes ;
la détermination de l'information relative à la pente de la sonde ultrasonique basée sur les pentes calculées de la sonde ultrasonique; et
la détermination de l'information relative à l'amplitude de la contrainte incluant l'information relative à l'amplitude de la contrainte, l'information relative à la période d'application de la contrainte et l'information relative à la pente de la sonde ultrasonique.

11. Support lisible par ordinateur comportant des instructions pouvant être exécutées par un ordinateur, configuré pour accomplir les actions suivantes :
a) la transmission/ réception de signaux ultrasoniques vers/de la part d'un objet cible au moyen d'une sonde ultrasonique pendant l'application d'une contrainte sur l'objet cible pour obtenir des premières données ultrasoniques ;
b) la transmission/ réception de signaux ultrasoniques vers/de la part d'un objet cible au moyen d'une sonde ultrasonique pendant le relâchement de la contrainte appliquée sur l'objet cible pour obtenir des secondes données ultrasoniques,
c) le calcul des amplitudes de contraintes, des périodes d'application des contraintes représentant des durées de l'application de contraintes et du relâchement, et des pentes de la sonde ultrasonique représentant les pentes de la sonde ultrasonique basées sur les différences de déplacements entre les déplacements correspondant aux lignes de balayage voisines,
d) la détermination des informations de contrainte basées sur les amplitudes de contraintes, les périodes d'application de contraintes et les pentes de sonde ultrasonique, dans lequel, les informations de contraintes englobent les informations d'amplitude de contraintes correspondant aux amplitudes des contraintes, les informations de périodes d'application des contraintes, correspondant aux périodes d'application de contraintes et les informations de pente de la sonde correspondant à la pente de la sonde ultrasonique; et
e) l'affichage de l'information relative à la contrainte sur l'unité d'affichage.

12. Support lisible par ordinateur selon la revendication 11, dans lequel l'information relative à la contrainte comprend en outre au moins un symbole, un graphique, une valeur numérique, un texte et des couleurs.

13. Support lisible par ordinateur selon la revendication 11, comportant en outre les instructions pouvant être exécutées par un ordinateur, de détection des amplitudes de contraintes basées sur les signaux captés relatifs à l'amplitude des contraintes.

14. Support lisible par ordinateur selon la revendication 13, dans lequel l'action consiste à :
calculer les amplitudes de contraintes basées sur les signaux d'amplitude de contraintes captés ; et
calculer les déplacements basés sur les premières et secondes données ultrasoniques, et
dans lequel l'action comprend :
la détermination de l'information relative à l'amplitude de la contrainte basée sur les amplitudes de contraintes calculées;
la détermination de l'information relative à la période d'application de la contrainte basée sur les périodes d'applications des contraintes calculées ;
la détermination de l'information relative à la pente de la sonde ultrasonique, basée sur les pentes calculées de la sonde ultrasonique ; et
la détermination de l'information relative à la contrainte incluant l'information relative l'amplitude de la contrainte, la période d'application de la contrainte et l'information relative à la pente de la sonde ultrasonique.
